# EUROPEAN PATENT APPLICATION

(11) **EP 4 714 427 A1**
(43) Date of publication of application: **25.03.2026**
(21) Application number: 24807054.2
(22) Date of filing: 01.05.2024
(51) Int. Cl.: A61K 8/49, A61K 8/34, A61K 8/37, A61K 31/22, A61K 31/045, A61K 31/404, A61P 17/00, A61P 43/00, A61Q 19/08, C07D 209/08, C12N 15/09

(54) **ANTI-WRINKLE AGENT**

(30) Priority: 15.05.2023 JP 2023080172
(71) Applicant: Shiseido Company, Ltd., Chuo-ku Tokyo 104-0061 (JP)
(72) Inventor: NISHIZUKA, Taichi, Tokyo 104-0061 (JP); NAKANISHI, Shinobu, Tokyo 104-0061 (JP); YANG, Shengfan, Tokyo 104-0061 (JP)
(74) Representative: Ter Meer Steinmeister & Partner
(86) International application number: PCT/JP2024/016756
(87) International publication number: WO 2024/237100

(57) **Abstract**

A wrinkle-improving agent is provided. A wrinkle-improving agent including citronellol, indole, farnesol, or amyl butyrate as an active component is provided.

## Description

### FIELD

The present invention relates to a wrinkle-improving agent comprising citronellol, indole, farnesol, or amyl butyrate as an active component.

### BACKGROUND

Wrinkles are commonly seen with aging, and it has been thought that one of the major factors of wrinkles is a decrease in collagen and elastin in the dermis. A number of anti-wrinkle agents have been reported, and their examples include nicotinic acid amide (such as Japanese Unexamined Patent Publication (Kokai) No. 2023-001403) and a *Schizonepeta* extract (Japanese Unexamined Patent Publication (Kokai) No. 2022-191664).

On the other hand, in recent years, olfactory receptors are known to be expressed in various organs (NPL 1). It has also been shown that olfactory receptors are expressed in fibroblasts, and that they are possibly involved in the collagen metabolism (NPL 2). For example, PTL 1 reports that geraniol, which is a rose aroma component, exerts an effect that promotes the production of collagen and elastin.

### [CITATION LIST]

### [PATENT LITERATURE]

[PTL 1] Japanese Unexamined Patent Publication (Kokai) No. 2022-130050

### [NON-PATENT LITERATURE]

[NPL 1] Pharmaceutics. 2021 Aug 23; 13(8): 1314
[NPL 2] Int J Mol Sci. 2021 Aug 27; 22(17): 9273

### SUMMARY

### [TECHNICAL PROBLEM]

The present invention aims to obtain a wrinkle-improving agent.

### [SOLUTION TO PROBLEM]

The present inventors intensively studied focusing on fragrance components to discover that citronellol, farnesol, and amyl butyrate have an effect to improve wrinkles, thereby reaching the present invention.

The present invention relates to the following.
[1] A wrinkle-improving agent comprising one or more selected from citronellol, indole, farnesol, and amyl butyrate as active components.
[2] An MMP-1 expression inhibitor comprising one or more selected from citronellol, indole, farnesol, and amyl butyrate as active components.
[3] An NEP expression inhibitor comprising one or more selected from citronellol, indole, farnesol, and amyl butyrate as active components.
[4] A collagen degradation inhibitor comprising one or more selected from citronellol, indole, farnesol, and amyl butyrate as active components.
[5] A collagen increasing agent comprising citronellol as an active component.

### [ADVANTAGEOUS EFFECTS OF INVENTION]

By the application of citronellol, indole, farnesol, or amyl butyrate, a wrinkle-improving-agent effect can be expected.

### DESCRIPTION OF EMBODIMENTS

Indole (CAS number: 120-72-9) is naturally present in plant and animal cells, and has an odor such as an animal odor, a foul odor, or an earthy odor. On the other hand, indole is also present in the flowers of gardenia, lily, and the like, and is also used for perfumes.

Farnesol (CAS number: 4602-84-0) is naturally occurring, and was found as a plant aromatic substance or an insect pheromone. It is an aroma component present in the flowers of rose, jasmine, and the like.

Amyl butyrate (CAS number: 540-18-1) is an ester, and has a banana-like aroma. It is an aroma component present also in fruits such as apples and strawberries.

Citronellol (CAS number: 106-22-9) is an aroma component contained in essential oils such as geranium oil and citronella oil, and has a rose-like aroma.

As mentioned above, it has been shown that olfactory receptors are also expressed in fibroblasts, and that they are possibly involved in the collagen metabolism. Geraniol has been reported to exert an effect that promotes the production of collagen and elastin. In view of this, the present inventors intensively studied focusing on fragrance components to discover that citronellol, farnesol, and amyl butyrate have an effect to improve wrinkles.

Thus, the present invention provides a wrinkle-improving agent comprising one or more selected from citronellol, indole, farnesol, and amyl butyrate as active components.

The improvement of wrinkles can be confirmed, for example, by measuring a decrease in the gene expression level or the protein production level of MMP-1, NEP, or the like, or by measuring inhibition of a decrease, or measuring an increase, in the production level of a protein involved in improvement of wrinkles, such as collagen or elastin, in a biological sample such as skin fibroblasts. Measurement of gene expression levels can be carried out by known methods in the present technical field, such as quantitative PCR and Northern blotting. For example, probes targeting mRNA of MMP-1 and/or NEP may be used. Protein levels can be measured using known methods in the present technical field, such as Western blotting, immunostaining, ICM, and ELISA. However, in addition to the methods described above, the wrinkle improvement can be confirmed by any method including visual inspection, measurement of the lengths and the number of wrinkles, and measurement of specific proteins such as collagen and elastin. For example, the production levels of the proteins such as collagen and elastin can be confirmed by measuring, by ELISA, the production levels of these proteins in skin samples such as skin models, or skin cells such as fibroblasts.

The present invention also provides an MMP-1 gene expression promoter, an NEP gene expression inhibitor, a collagen degradation inhibitor, and a collagen increasing agent, comprising one or more selected from citronellol, indole, farnesol, and amyl butyrate as active components. In one aspect, these agents act in skin fibroblasts.

MMP-1 (matrix metallopeptidase-1) is a gene encoding a collagen-degrading enzyme. NEP (neutral endopeptidase) is a gene encoding an elastin-degrading enzyme. Decreased production of MMP-1 leads to retention of tissue collagen, resulting in inhibition of wrinkle formation. Further, decreased production of NEP leads to retention of tissue elastin, resulting in inhibition of wrinkle formation.

Measurement of gene expression, as stated above, can be carried out by known methods in the present technical field, such as quantitative PCR and Northern blotting. Inhibition of gene expression, for example, means a decrease that is statistically significant (for example, Student's t-test) at a significance level of 5%, and/or a decrease of, for example, 10% or greater, 20% or greater, 30% or greater, 40% or greater, 50% or greater, 60% or greater, 70% or greater, 80% or greater, 90% or greater, or 100%.

Measurement of the protein production level, as stated above, can be carried out by known methods in the present technical field, such as Western blotting, immunostaining, ICM, and ELISA. Promotion of the protein production level, in one embodiment, means an increase that is statistically significant (for example, Student's t-test or Dunnett's test) at a significance level of 5%, and/or an increase of, for example, 1% or greater, 2% or greater, 3% or greater, 4% or greater, 5% or greater, 10% or greater, 20% or greater, 30% or greater, 40% or greater, 50% or greater, 60% or greater, 70% or greater, 80% or greater, 90% or greater, 100% or greater, or even greater. Inhibition of the protein production level, for example, means a decrease that is statistically significant (for example, Student's t-test or Dunnett's test) at a significance level of 5%, and/or a decrease of, for example, 1% or greater, 2% or greater, 3% or greater, 4% or greater, 5% or greater, 10% or greater, 20% or greater, 30% or greater, 40% or greater, 50% or greater, 60% or greater, 70% or greater, 80% or greater, 90% or greater, or 100%.

Collagen degradation-inhibiting effect can be measured by measuring the collagen production rate by known methods in the present technical field such as ELISA, for example, sandwich ELISA. In one embodiment, the collagen degradation-inhibiting effect can be judged to be present when, for example, there is no statistically significant decrease in the collagen production rate at a significance level of 5% (for example, Student's t-test or Dunnett's test), or when, for example, there is no decrease in the collagen production rate by 1% or greater, 2% or greater, 3% or greater, 4% or greater, or 5% or greater. Alternatively, the collagen degradation-inhibiting effect can be measured by measuring the production level of a gene or protein involved in collagen degradation. In one embodiment, when MMP-1 shows inhibition of gene expression within the range described above, the collagen degradation-inhibiting effect can be judged to be present.

Collagen increasing effect can be measured by measuring the collagen production rate by known methods in the present technical field such as ELISA, for example, sandwich ELISA. In one embodiment, the presence of the collagen increasing effect can be judged to be present when, for example, the collagen production rate shows an increase that is statistically significant (for example, Student's t-test or Dunnett's test) at a significance level of 5%, or an increase of, for example, 1% or greater, 2% or greater, 3% or greater, 4% or greater, 5% or greater, 10% or greater, 20% or greater, 30% or greater, 40% or greater, 50% or greater, or even greater.

The biological sample may be a sample in which the wrinkle-improving effect can be measured, or a cell culture in which the expression levels of genes such as MMP-1 and NEP, the protein production levels corresponding to these genes, or the production levels of proteins involved in wrinkle improvement such as collagen and elastin can be measured. For example, keratinocytes or fibroblasts, especially fibroblasts, can be preferably used. Alternatively, the biological sample may be a skin sample, or a 3D-constructed cultured skin model may be used. The cell may be derived from any animal, but from the viewpoint of development of cosmetics and pharmaceuticals, is preferably derived from a human.

The present invention also provides a method for improving wrinkles in a subject, comprising applying citronellol, indole, farnesol, or amyl butyrate, or the agent of the present invention, or a composition comprising the same.

The subject to whom the method of the present invention is applied include, for example, subjects who wish to prevent or improve wrinkles, subjects who have been determined to have many or deep wrinkles, subjects who are deficient in collagen or elastin, and subjects with increased gene expression levels or protein production levels of MMP-1, NEP, and the like.

The method according to the present application is for cosmetic purposes and may exclude medical acts carried out by a physician or medical professional. In addition, the method according to the present application may be a method for supporting a cosmetic act of a subject.

The present invention also provides a composition for improving wrinkles, comprising citronellol, indole, farnesol, or amyl butyrate, or the agent of the present invention. The composition may be a cosmetic, a pharmaceutical, or a quasi-drug.

For the application, the composition can be applied via any route, such as transdermal, oral, transmucosal, nasal, intravenous, intraarterial, or subcutaneous, but from the viewpoint of having an effect on the skin, is preferably applied transdermally, i.e., to the skin. For the skin, the composition can be applied to any part thereof, such as the face, head, neck, limbs, or torso.

In the case of a cosmetic, the composition can be blended into a face or body cosmetic such as a toner, an emulsion, a serum, a cream, a lotion, a pack, an essence, or a gel; a makeup cosmetic such as a foundation, a cosmetic primer, or a concealer; or even a bath additive. In the case of a pharmaceutical for transdermal administration, the composition can be formulated into an external skin preparation. The form needs only to be applicable to the skin and is not particularly limited. For example, any dosage formulation can be applied, such as a solution, an emulsion, a solid, a semi-solid, a powder, a powder dispersion, a water-oil bilayer separation, a water-oil-powder trilayer separation, an ointment, a gel, an aerosol, a mousse, or a stick. In addition, bases and excipients commonly used in cosmetics and external skin preparations, such as preservatives, emulsifiers, and pH adjusters, may be used.

The amount of active component in the agent or composition of the present invention can be arbitrarily selected from the viewpoint of exhibiting a wrinkle-improving effect. For example, citronellol, indole, farnesol, or amyl butyrate may be included at 0.0005 to 100.0 mM. From the viewpoint of sufficiently exhibiting the effect, preferably 0.005 mM or more, more preferably 0.05 mM or more can be blended. On the other hand, since citronellol, indole, farnesol, and amyl butyrate have specific odors, from the viewpoint of avoiding an excessively strong odor, preferably 10.0 mM or less can be blended, and more preferably 5.0 mM or less may be blended. The components described above may be combined in any ratio, and in that case, the total amount of the components is preferably within the above range.

The present invention also provides citronellol, indole, farnesol, or amyl butyrate for wrinkle improvement, wherein the wrinkle improvement is preferably mediated by inhibition of MMP-1 and/or NEP gene expression. In one aspect, the wrinkle improvement is wrinkle improvement of the dermis, and is exhibited by a decrease in a collagen-degrading enzyme and/or an elastin-degrading enzyme caused by inhibition of gene expression or protein production of MMP-1 and/or NEP in skin fibroblasts. In one aspect, the wrinkle improvement is wrinkle improvement of the dermis, and is not accompanied by a decrease, or is accompanied by an increase, in the collagen production level in skin fibroblasts. In one example of such an aspect, the collagen degradation-inhibiting effect of citronellol, indole, farnesol, or amyl butyrate does not cause a decrease in the collagen production level. In one example of such an aspect, citronellol, indole, farnesol, or amyl butyrate causes an increase in collagen. In one aspect, the inhibition of collagen degradation is exhibited by inhibiting gene expression or protein production of MMP-1.

The present invention also provides a use of citronellol, indole, farnesol, or amyl butyrate in the manufacture of a wrinkle-improving agent.

All literatures mentioned in the present specification are incorporated herein by reference in the entirety thereof.

The Examples of the present invention described below are intended for exemplification only, and do not limit the technical scope of the present invention. The technical scope of the present invention is limited only by the description of the claims. Modifications to the present invention, for example, addition, deletion, and substitution of constituent elements of the present invention, can be made without departing from the spirit of the present invention.

### EXAMPLES

To confirm wrinkle-improving effects of citronellol, indole, farnesol, and amyl butyrate, expressions of genes involved in their functions were examined by analysis. More specifically, expression levels of MMP-1 and NEP were measured according to the methods described below.

### Example 1: Effect of citronellol, indole, farnesol, or amyl butyrate on keratinocyte.

### 1-1: Cell culture

Cells of the human neonatal dermal fibroblast cell line NB1RGB (RIKEN BRC, Japan) were seeded in Dulbecco's Modified Eagle Medium (DMEM, Cat No. 043-30085, Wako, Japan) comprising 10.0% (v/v) Fetal Bovine Serum (FBS, Cat No. SH30071.03, Hyclone, UK) and 1.0% (v/v) antifungal agent (Antibiotic-Antimycotic 100X, Cat No. 15240-062, Invitrogen, USA) in a 24-well plate (Cat No. 3526, Corning, USA) at a density of 10.0 × 10⁴ cells/well and cultured within a CO₂ incubator (CO₂ concentration of 5%, 37°C) for 24 h. The medium was removed and then replaced with a medium to which indole (CAS number: 120-72-9, Wako Pure Chemical Industries, Ltd.), farnesol (CAS number: 4602-84-0, Wako Pure Chemical Industries, Ltd.), amyl butyrate (CAS number: 540-18-1, Wako Pure Chemical Industries, Ltd.), or citronellol (CAS number: 106-22-9, Wako Pure Chemical Industries, Ltd.) was added to a final concentration of 0.05 mM, and the cells were further cultured within the CO₂ incubator for 48 h. As the control, a medium not comprising these components was used.

### 1-2: RNA extraction and purification, quantification, and purity measurement

RNA extraction and purification were carried out using a PureLink^{™} RNA Mini Kit (Cat No. 12183018A, Invitrogen, USA), a portion of the purified RNA was aliquoted into a UV-transparent 96-well plate and diluted 10-fold with Tris-EDTA Buffer, and absorbances (OD230, OD260, and OD280) at 230 nm, 260 nm, and 280 nm were measured using a microplate reader (SPARK (registered trademark), 10M TECAN, Switzerland). RNA concentrations were calculated using OD260 and diluted with TE Buffer to adjust the RNA concentrations to 10 µg/mL.

### 1-3: Gene expression analysis by real-time PCR method

Reverse transcription of RNA was carried out using SuperScript^{™} IV VILO^{™} Master Mix with ezDNase (Cat No. 11766050, Invitrogen, USA). 4 µL of SuperScript^{™} IV VILO^{™} Master Mix and 6 µL of Nuclease-free Water were added per well in an 8-tube strip and heated at 25°C for 10 min, 50°C for 10 min, and 85°C for 5 min using real-time PCR (QuantStudio (registered trademark) 3, Applied Biosystems, USA) to synthesize cDNA. 10 µL of TaqMan (registered trademark) Fast Advanced Master Mix (Cat No. 4444557, Applied Biosystems, USA), 1 µL of TaqMan Gene Expressior, 7 µL of UltraPure^{™} Distilled Water (Invitrogen, Cat No. 10977-015, USA), and 2 µL of cDNA were added per well in a PCR plate and sealed. A primer (Hs00899658_m1) for MMP-1 and a primer (Hs00153510_m1) for NEP; and a primer (Hs02786624_g1) for GAPDH as an internal standard gene; were used to carry out real-time qPCR, the threshold cycle (Ct) value of each of the MMP-1 gene and the NEP gene in the medium supplemented with each component was calculated, and then Ct value was corrected with GAPDH to obtain a ΔCt value. Assuming that gene level doubles per cycle, when gene expression level of the control was set at 1, the gene expression level in the medium supplemented with each component was determined. In the analysis of gene expression level, the average value of 24-well plate × 3 wells was used for each treatment group.

Results are shown in Table 1 below.

**[Table 1]**

| Table 1: Promotion of MMP-1 expression by addition of each component | | | | |
|---|---|---|---|---|
| Substance name | Indole | Farnesol | Amyl butyrate | Citronellol |
| Gene expression level | 0.36 | 0.22 | 0.24 | 0.25 |
| SD | 0.04 | 0.00 | 0.03 | 0.02 |
| p | <0.01 | <0.01 | <0.05 | <0.01 |

| | | | | |
|---|---|---|---|---|
| (Comparison with the no-addition group by paired t-test (two-tailed); expressed as the ratios taking the value in the no-addition group as 1) | | | | |

**[Table 2]**

| Table 2: Promotion of NEP expression by addition of each component | | | | |
|---|---|---|---|---|
| Substance name | Indole | Farnesol | Amyl butyrate | Citronellol |
| Gene expression level | 0.40 | 0.44 | 0.43 | 0.85 |
| SD | 0.02 | 0.01 | 0.03 | 0.05 |
| p | <0.01 | <0.01 | <0.01 | Downward trend |

| | | | | |
|---|---|---|---|---|
| (Comparison with the no-addition group by paired t-test (two-tailed); expressed as the ratios taking the value in the no-addition group as 1) | | | | |

As shown in Tables 1 and 2, the addition of indole, farnesol, amyl butyrate, and citronellol to the dermal fibroblasts resulted in decreases in the expression of MMP-1 and NEP.

### Example 2: Effect of citronellol on collagen level

In the present Example, changes in the collagen production level in dermal fibroblasts were investigated for citronellol, which showed an inhibitory effect on the expression of the MMP-1 gene in the dermal fibroblasts in Example 1.

### 2-1: Cell culture

The same NB1RGB cells as in Example 1 were seeded in Eagle's Minimal Essential Medium (EMEM, Cat No. 051-07615, Wako, Japan) comprising 10.0% (v/v) Fetal Bovine Serum (FBS, Cat No. SH30071.03, Hyclone, UK) and 1.0% (v/v) antifungal agent (Antibiotic-Antimycotic 100X, Cat No. 15240-062, Invitrogen, USA) in a 24-well plate (Cat No. 3526, Corning, USA) at a density of 10.0 × 10⁴ cells/well and cultured within a CO₂ incubator (CO₂ concentration of 5%, 37°C) for 24 h. After 24 h, the medium was removed and replaced with a medium to which citronellol (CAS number: 106-22-9, Wako Pure Chemical Industries, Ltd.) was added so as to have the final concentration shown in Table 3 below, and the cells were further cultured within the CO₂ incubator for 48 h. As the control, a medium not comprising citronellol was used.

### 2-2: Cell-activating effect evaluation

According to the following method, the effect of citronellol on proliferation of dermal fibroblasts was evaluated.

A 96-well plate from which the medium was removed was washed with 100 µL of PBS(-). 100 µL of 0.5 mg/mL 3-(4,5-dimethylthiazol-2-yl)-2,5-diphenyltetrazolium bromide (MTT, CAS No. 298-93-1, Sigma-Aldrich, USA) solution was then added and incubated within a CO₂ incubator for 2 h. After removing the MTT solution and washing with 100 µL of PBS(-), 200 µL of 2-propanol (CAS No. 67-63-0, Wako, Japan) was added to dissolve the insoluble formazan. A dye was uniformly dispersed within the 96-well plate, and then absorbance (OD570) at 570 nm was measured using a microplate reader (SPARK (registered trademark), 10M TECAN, Switzerland). Setting OD570 of the control group at 100%, OD570 of the citronellol-added group, i.e., the effect on cell proliferation, was calculated as the cell-activating effect (%) of citronellol. In the analysis of cell-activating effect, the average value of 96-well plate 9 × 3 wells was used for each treatment group.

### 2-3: Measurement of collagen

A supernatant of the culture cultured in 2-1 was dispensed into a new 96-well plate and frozen for storage (-80°C). The collagen production rate in the culture supernatant was measured by sandwich ELISA, and the effect of citronellol on the collagen level was evaluated.

100 µL of culture supernatant diluted 4-fold with PBS(-) was added to a high-binding 96-well plate, and incubated overnight at 4°C. Type I collagen solution (Cat No. 009-001-103, RCK, USA) was used as a standard substance. After removing the immobilized culture supernatant and washing of the microplate with 200 µL of PBS(-) containing 0.05% Tween (registered trademark) 20 (PBS-T, Tween (registered trademark) 20: CAS No. 9005-64-5, Sigma-Aldrich, USA), 150 µL of 1% Bovine Serum Albumin (BSA, Cat No. PRL68700-50G, Proliant, USA) solution was added to the microplate, and incubation was performed at 37°C for 1 hour. The BSA solution was removed, and the microplate was washed with 200 µL of PBS-T, followed by adding 100 µL of 100 ng/mL biotin-labeled Collagen Type I Antibody (Cat. No. 600-406-103, ROCKLAND, USA) solution prepared with PBS (-) containing 0.5% BSA, and performing incubation at 37°C for 1 hour. After removing the antibody solution, the microplate was washed with 200 µL of PBS-T, and then 100 µL of Streptavidin-HRP (Cat No. CJ30H-1, Agilent Technologies, USA, 1:10,000) solution prepared with PBS (-) containing 0.5% BSA was added thereto, followed by performing incubation at room temperature for 30 minutes. The Streptavidin-HRP solution was removed, and the microplate was washed with 200 µL of PBS-T, followed by adding 100 µL of 2,2'-azinobis(3-ethylbenzothiazoline-6-sulfonic acid) diammonium salt (ABTS, Cat No. 5110-0010, KPL, USA) solution thereto and confirming color development. The dye was uniformized within the 96-well plate, and then absorbance (OD405) at 405 nm was measured using a microplate reader.

Setting OD405 of the control group at 100%, the collagen production rate of citronellol was calculated. In addition, the OD405 of the control and citronellol were each divided by OD570 measured in 2-2 to calculate the collagen production rate per cell. Setting the collagen production rate per cell in the control group at 100%, the collagen production rate of citronellol per cell was calculated.

The results of 2-2 confirmed that no significant increase or significant decrease in proliferation of fibroblasts was observed, and citronellol had no cell-activating effect or toxicity. The results of 2-3 are shown in Table 3 as the collagen production rate due to citronellol when collagen production rate of the control was set at 100%.

**[Table 3]**

| Table 3: Collagen production rate (%) per cell with addition of citronellol | | |
|---|---|---|
| Substance name | No addition of Citronellol | 50 µM Citronellol |
| Gene expression level | 100.0 | 102.4 |
| SD | 1.2 | 0.7 |
| p | | p<0.05 |

| | | |
|---|---|---|
| (Comparison with the no-addition group by unpaired t-test (two-tailed); expressed as the ratio taking the value in the no-addition group as 100) | | |

According to Table 3, it was confirmed that, due to the addition of citronellol, the collagen production rate per cell was significantly increased. These results indicate that citronellol increases the collagen production level in fibroblasts. When considering the results of Example 1, it was suggested that, in the collagen increasing effect of citronellol, the collagen degradation-inhibiting effect by the inhibition of MMP-1 expression may be involved.

Based on these results, citronellol, indole, farnesol, and amyl butyrate are expected to inhibit the expression of MMP-1 and NEP, thereby decreasing a collagen-degrading enzyme and an elastin-degrading enzyme, or increasing collagen, to produce a favorable effect on wrinkle improvement.

## Claims

1. A wrinkle-improving agent comprising one or more selected from citronellol, indole, farnesol, and amyl butyrate as active components.

2. An MMP-1 expression inhibitor comprising one or more selected from citronellol, indole, farnesol, and amyl butyrate as active components.

3. An NEP expression inhibitor comprising one or more selected from citronellol, indole, farnesol, and amyl butyrate as active components.

4. A collagen degradation inhibitor comprising one or more selected from citronellol, indole, farnesol, and amyl butyrate as active components.

5. A collagen increasing agent comprising citronellol as an active component.
